# EUROPEAN PATENT SPECIFICATION

(11) **EP 2 558 568 B1**
(45) Date of publication and mention of the grant of the patent: **17.10.2018**
(21) Application number: 11723646.3
(22) Date of filing: 14.04.2011
(51) Int. Cl.: C12N 5/00, C12M 1/02, C12M 3/08, C12N 5/0775

(54) **METHOD FOR OBTAINING A POPULATION OF STROMAL PROGENITOR CELLS**
VERFAHREN ZUR GEWINNUNG EINER POPULATION VON VORLAÜFERZELLEN
PROCÉDÉ D'OBTENTION D'UNE POPULATION DE CELLULES PROGENITRICES

(30) Priority: 14.04.2010 IT MO20100111
(43) Date of publication of application: 20.02.2013
(73) Proprietor: Rigenerand S.r.l., 41036 Medolla (MO) (IT)
(72) Inventor: Dominici, Massimo, 44100 Ferrara (FE) (IT); Cafarelli, Luigi, 41125 Modena (MO) (IT); Veronesi, Elena, 41035 Finale Emilia (MO) (IT); Piccinno, Maria Serena, 41121 Modena (MO) (IT); Paolucci, Paolo, 40138 Bologna (BO) (IT); De Santis, Giorgio, 41121 Modena (MO) (IT); Pierfranco, Conte, 56127 Pisa (PI) (IT)
(74) Representative: Bergamini, Silvio
(86) International application number: PCT/IB2011/051614
(87) International publication number: WO 2011/128868

(56) References cited:
- WO-A1-2009/027563
- US-A1- 2005 008 626
- US-A1- 2005 076 396
- ZUK P A ET AL: "Multilineage cells from human adipose tissue: Implications for cell-based therapies", TISSUE ENGINEERING, LARCHMONT, NY, US LNKD- DOI:10.1089/107632701300062859, vol. 7, no. 2, 1 April 2001 (2001-04-01), pages 211-228, XP002198710, ISSN: 1076-3279

## Description

### Field of the invention

The present invention relates to a method of obtaining a population of cells, particularly a population of stromal progenitor cells from a quantity of adipose tissue collected from a living being.

### Background art

The term stromal progenitors, hereinafter briefly referred to as SP, defines all cell populations that are capable of proliferation and differentiation, and also provide support for surrounding tissues and cells.

The SPs are rare cell elements located in the tissues of living beings, which are designed to perpetuate their function by turnover of damaged and/or senescent cells.

They were initially found in high-turnover tissues, such as the hematopoietic and epithelial system, but can be found, although less frequently, in tissues and organs having little or no regenerative capacity, such as the central nervous system.

Originally, bone marrow studies started more than thirty years above could identify certain SP that will be referred to hereinafter as mesenchymal SP, which are capable of maintaining hemopoiesis and osteogenesis, thereby providing functional and structural support.

In vitro studies showed that these cells have a high proliferative potential, as well as differentiating capacities, in certain appropriate conditions, i.e. the ability of converting into cell elements belonging to bone, cartilage, adipose, muscular and nervous tissue.

This knowledge concerning the characteristics of bone marrow mesenchymal SP have extended the scope of research to introduce novel therapies in the so-called regenerative medicine.

Due to their differentiating potential, bone marrow mesenchymal SP have been studied for regeneration of injured tissues after trauma and acute and chronic degenerative events, such as cardiopathies; in oncology, they may be used to carry drugs having an antitumor action and also find application in autoimmune diseases, due to the production of immune response modulating molecules. Furthermore, due to their support function, they have been found to be useful as an aid in hemopoietic stem cell transplantation.

Some of these studies led to clinical applications of SP, such as myocardial infarct, diabetes mellitus, autoimmune diseases, bone regeneration, burns, lipodystrophies and liver failure.

In all the above indications, and for effective therapeutic application, a great number of SP needs to be infused or transplanted, i.e. of the order of various millions per kilogram weight of a patient.

This requires prolonged expansion of SP outside the donor, i.e. in vitro in culture flasks.

This is required because of the poor presence of SP in the original bone marrow tissue, and may be a limitation.

Furthermore, the collection site (the bone marrow) may not be easily accessible and be damaged due to the presence of neoplastic cells or simultaneous pharmacological treatments.

Therefore, further SP collection sites have been considered, such as the periosteum, bone trabeculae, the skeletal muscle, the lung, the umbilical cord and particularly the subcutaneous adipose tissue (AT).

Concerning the AT, the main cell is the adipocyte, which is as large as about 100 µm and fulfills the main role of the AT, i.e. storing energy in the form of triglycerides introduced by the diet.

Also in the AT the SP are a pool of progenitors which replicate in response to appropriate hormone stimulation, thereby allowing part of the progenies to differentiate into mature adipocytes, and also act as a support to vascular structures, whereby they are defined as stromal pericytes.

The AT mass in adult humans is a function of the diet and life style and ranges from 2-3% the total weight in an athlete to 60-70% in an obese individual.

The increased occurrence of obesity has increased AT availability, also due to an increase in cosmetic surgery for reducing subcutaneous adipose mass for aesthetic purposes or else.

In these cases AT collection may currently occur by liposuction.

As a result, the AT is a potential source of SP, due to its abundance and accessibility.

Zuk PA et al, in the article "Human adipose tissue is a source of multipotent stem cells", published in Molecular Biology of The Cell (2002), started various studies to assess the analogy between SP of adipose and bone marrow origin. These initial tests showed a number of analogies in terms of differentiation potential and antigen expression, and this preliminary data allowed the introduction of SP in many fields of regenerative medicine for cardiology and cosmetic medicine applications, particularly starting from patient-derived, i.e. autologous cells.

According to patent applications WO2009027563 and EP 2184068 a "Population of adult stem cells derived from cardiac adipose tissue and use thereof in cardiac generation" is known.

These inventions relate to the isolation and characterization of a novel population of adult stem cells derived from fatty heart tissue which express GATA-4 and/or Cx43 in a constitutive manner. Said cell population can be used in cell therapy protocols in order to regenerate damaged myocardial tissue.

From patent application US2005008626 "Methods of using adipose tissue-derived cells in the treatment of cardiovascular conditions" is known.

According to this patent, cells present in processed lipo-aspirate tissue are used to treat patients, including patients with cardiovascular conditions, diseases or disorders. Methods of treating patients include processing adipose tissue to deliver a concentrated amount of stem cells obtained from the adipose tissue to a patient. The methods may be practiced in a closed system so that the stem cells are not exposed to an external environment prior to being administered to a patient. Accordingly, in a preferred method, cells present in processed lipo-aspirate are placed directly into a recipient along with such additives necessary to promote, engender or support a therapeutic cardiovascular benefit.

From patent application US2005076396 "Adipose-derived stem cells and lattices" are known.

This patent application provides adipose-derived stem cells (ADSCs), adipose-derived stem cell-enriched fractions (ADSC-EF) and adipose-derived lattices, alone and combined with the ADSCs of the invention. In one aspect, the patent application provides an ADSC substantially free of adipocytes and red blood cells and clonal populations of connective tissue stem cells. The ADSCs can be employed alone or within biologically-compatible compositions, to generate differentiated tissues and structures, both in vivo and in vitro. Additionally, the ADSCs can be expanded and cultured to produce molecules such as hormones, and to provide conditioned culture media for supporting the growth and expansion of other cell populations. In another aspect, the patent application provides a adipose-derived lattice substantially devoid of cells, which include extracellular matrix material from adipose tissue. The lattice can be used as a substrate to facilitate the growth and differentiation of cells, whether in vivo and in vitro, into anlagen or even mature tissues or structures.

The present state of the art is limited in that large amounts of subcutaneous AT have to be collected to obtain an adequate number of SP. These volumes of collected fat are usually above 0.5 L and may be as large as 1 L. While these are large volumes in absolute terms, they have relatively little incidence on an obese or overweight patient, i.e. having a Body Mass Index (BMI) parameter exceeding 25. Such volumes cannot be obtained from low BMI individuals (having a BMI of less than 18.5) which might difficultly have the required amount of autologous SP.

Therefore, this prior art method is only applicable to a limited number of patients.

Furthermore, invasive surgical procedures are required, involving the hazards of any surgery, and particularly the occurrence of fat embolism.

Also, AT collections always require general anesthesia of the patient, with the care and the hazards involved thereby.

### Disclosure of the invention

It is an object of the present invention to improve the prior art.

Another object of the invention is to provide a method of obtaining a population of cells that allows the therapeutic application of AT-derived SP to be extended to a greater number of individuals.

A further object of the invention is to provide a method of obtaining SP from a small AT mass, i.e. of the order of a few hundredths to thousandths of milliliter, which is present and easily collectable in all types of patients, even in very thin individuals (with a BMI of less than 18.5).

Yet another object of the invention is to collect sufficient amounts of SP from the AT under local anesthesia.

In one aspect, the invention relates to a method of obtaining a population of SP cells as defined in claim 1.

Therefore, the invention provides the following advantages:
collecting an adequate number of SP from a very small volume of AT in patients possibly having a BMI of less than 18.5;
obtaining considerable amounts of cells from very small amounts of tissue collected from a patient;
obtaining the small amounts of collected tissue by minimally invasive surgery, under local anesthesia, with considerably reduced hazard, discomfort and pain for the patient;
using small amounts of AT to isolate a population of SP that maintains the properties of its progenitors, i.e. immunogen characteristics and differentiation capacities;
obtaining progenitor cells from patients for further transplantation, to regenerate damaged tissues.

### Brief description of the drawings

Further features and advantages of the invention will be more readily apparent upon reading of the detailed description of a preferred not exclusive embodiment of a method of obtaining a population of cells, particularly SP cells from AT, which is shown as a not limiting example by the annexed drawings, in which:
FIG. 1 is a view of the lipo-suctioned sample divided into 9 parts, each having a volume of about 0.020-0.025 mL and undergoing three different procedures in triplicate, namely the parts designated as sample E underwent an enzyme digestion process, the three parts designated as sample M underwent a mechanical digestion process and the three parts designated as sample P were cultivated;
FIG. 2a is a view of the skeleton of the adipose tissue in the supernatant after enzyme digestion, obtained using an inverted microscope (at x-100 magnification);
FIG. 2b is a diagram that shows the number of cells (in millions) obtained after digestion of samples E and M;
FIGS. 3a and 3b are two images of an in vitro culture of sample P, obtained at zero days and seven days respectively, particularly FIG. 3a is a view of the adipose mass, FIG. 3b is a view of the cells adhered to the plastic of a culture flask or container and defined as pre-adipocytes (zone 1).
FIG. 3c is an image of the 10-days in vitro culture of a sample E, which shows the presence of both adherent pre-adipocytes and SP isolated from adipose tissue.
FIG. 3d is an image obtained using an inverted microscope (at x-100 magnification) of the 10-days in vitro culture of a sample M where it can be noted that the simple culture process is not effective in SP isolation, as no cells are shown to adhere to the culture flask or container;
FIG. 4 is a diagram that shows the growth of sample E in which SP cell doubling is shown in the first four culture passages;
FIG. 5a is an image of the SP of sample E at culture passage four;
FIG. 5b is an image of SP showing negativity for staining of typically intra-cytoplasmic fat droplets, known as Oil-Red O;
FIG. 5c is an image of the pre-adipocytes isolated from sample P, showing positivity for Oil-Red-O staining;
FIG. 6a is a diagram showing the expression of the CD45 antigen;
FIG. 6b is a diagram showing the expression of the CD31 antigen;
FIG. 6c is a diagram showing the expression of the CD146 pericyte antigen;
FIG. 6d is a diagram showing the expression of the CD90 antigen;
FIG. 6e is a diagram showing the expression of the CD73 antigen;
FIG. 6f is a diagram showing the expression of the CD105 antigen;
FIG. 7a shows an image obtained using an inverted microscope of non osteogenically induced SP, negative for Alizarin RED staining;
FIG. 7b shows a x-100 magnified image of osteogenically induced SP, i.e. positive for Alizarin Red staining;
FIG. 7c shows a x-100 magnified image of non-adipogenically induced SP, i.e. negative for Oil-RED-O staining;
FIG. 7d shows a x-100 magnified image of adipogenically induced SP, i.e. positive for Oil-Red-O staining;
FIG. 7e shows a x-100 magnified image of non-chondrogenically induced SP, i.e. negative for Alcian Blue staining;
FIG. 7f shows a x-100 magnified image of chondrogenically induced SP, i.e. positive for Alcian Blue staining.

### Detailed description of a few preferred embodiments

### Example 1.

The AT sample was collected from the subcutaneous facial area of a healthy 46-year old female donor by Coleman liposuction. Other methods may be also used for collection.

The AT fragments were washed three times with a saline solution known as Dulbecco-phosphate buffer solution (D-PBS) added with antibiotic (1 U/mL penicillin, 1 mg/mL streptomycin and 2.5 mg/mL amphotericin B), for an overall time of 15 minutes.

The last washing step, which was designed to yield a sample with no liquid component, was carried out using a filter (100 µm cell strainer).

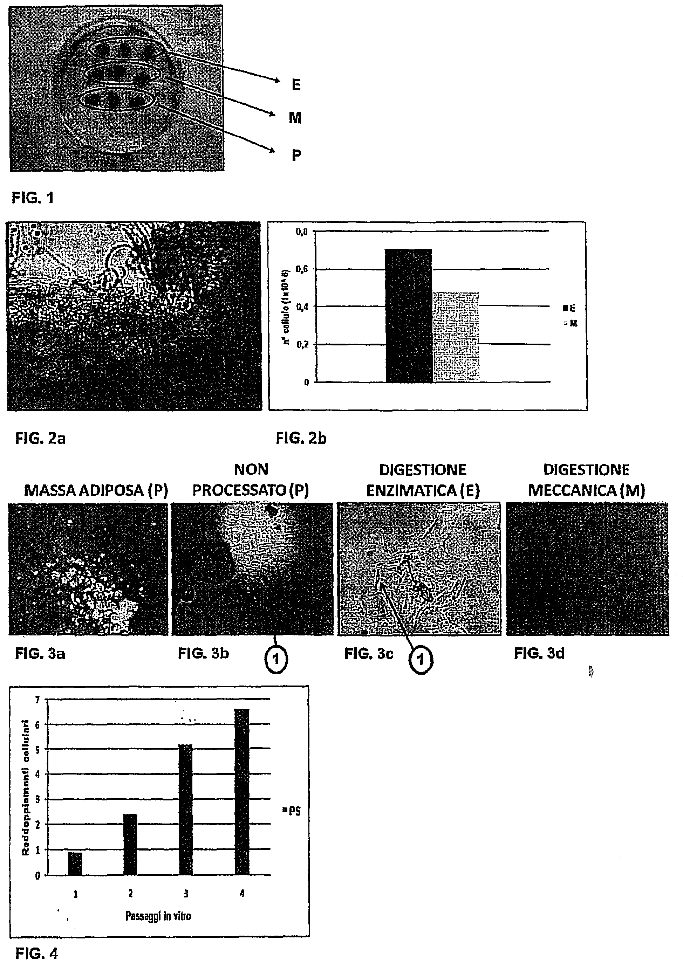

The AT fragments were transferred into a sterile container (petri dish) and divided into nine parts, each having a volume of about 0.025 mL. The latter, as shown in Figure 1, underwent three different procedures in triplicate: three parts (sample E) underwent the same enzyme digestion process, three parts (sample M) underwent the same mechanical digestion process and finally, the last three parts, designated as sample P were all cultivated.

Sample E was digested by the enzyme solution, consisting of the enzyme buffer added with the enzyme mixture, and at the same time sample M was mechanically digested, i.e. with the enzyme buffer only, and no enzyme mixture, and finally sample P was cultivated without being processed.

Samples E and M were fragmented using eye scissors. Sample E was incubated (at 37°C) with the enzyme solution, and sample M with the enzyme buffer, in a ratio of 0.004 mL AT per each mL of each solution.

The enzyme buffer is a medium generally known as Dulbecco's Modified Eagle Medium (DMEM), added with 1 U/mL penicillin, 1 mg/mL streptomycin, 1 mM sodium pyruvate, not essential amino acids (a solution composed of: L-Alanin (0.89 mg/dL); L-Asparagin H20 (1.5 mg/dL); L-Aspartic acid (1.33 mg/dL); L-Glutamic Acid (1.47 mg/dL); Glycin (0.75 mg/dL); L-Prolin (1.15 mg/dL); L-Serin (1.05 mg/dL).

The enzyme mixture, e.g. a mixture known as "COLLAGENASE P", sold by Roche, contains Clostripain in a concentration of 2.8 U/mg lyophilizate, Protease (Azocoll) in a concentration of 160 U/mg and Tripsin (BAEE) in a concentration of 0.23 U/mg.

Samples E and M were transferred into one or more 50 ml cylindrical containers known as Falcon.

The quantity of each sample in each container was about 30 ml; the AT samples were stirred at 37°C for 120 minutes, i.e. the ideal conditions for enzyme activity. Oscillation was longitudinally directed (75 oscillations/minute) in the direction of the longitudinal axis of the container. At the end of the incubation time, the samples were centrifuged at 1,500 rpm for 10 minutes, thereby yielding a supernatant, to be discarded, and a pellet composed of all AT cell elements. Referring to Figure 2a, observation of sample E with an optical microscope shows the presence of the skeleton of adipose tissue in the supernatant, confirming that such tissue was effectively digested by collagenase.

The pellet was washed two more times after filtration using a 100 µm cell strainer, to remove mature adipocytes.

Once the above process was completed, cell counting was effected by a cell survival test using 0.4% Trypan Blue staining. 701,000 cells and 482,000 cells were obtained on average for samples E and M respectively (FIG. 2b).

These cells were seeded with a density of 10,000/cm² in cell culture containers (flasks) with a Quantum 333 medium added with 1 U/mL penicillin and 1 mg/mL streptomycin.

Figures 3a and 3b show the in vitro culture of sample P at 0 days and 7 days respectively.

The unprocessed 0-day sample exhibits the adipocyte-rich adipose mass (FIG. 3a), while FIG. 3b shows, at 7 days, cell elements adhering to the plastic and arranged around the adipose mass.

These cell elements have an elongate shape and are characterized by the presence of small intra-cytoplasmic lipid vacuoles, and hence are called pre-adipocytes (zone A in FIG. 3b).

After 15 days, in spite of the great number of cells that were isolated initially, the culture of sample P showed a limited growth, no in vitro expansion being thus observed.

Conversely, after 10 days from the start of culture, sample E was found to contain about 50% pre-adipocytes and as many fibroblastoid elements in the flask (zone 1 of FIG. 3c).

Finally, Figure 3d shows sample M: no cell population could be isolated here, because no cells adhere to the culture flask.

The cultures so prepared were continued in the incubator at a controlled atmosphere (37°C, 5% CO2) and the medium was replaced every 2-3 days to full flask growth, which was achieved by sample E only.

Later steps involved trypsinization with a trypsin-EDTA solution (0.05%-0.02%) and reseeding in new flasks with a density of 5000 cells/cm2.

After culture passage four, 50 x 10⁶ cells were obtained in sample E, whereas no growth was observed in the other groups. Cell doubling was calculated with the formula log(N1/N2)/log2, where N1 e N2 are the cell count at the i^{th} passage and at the i+1^{th} passage (FIG. 4). In vitro growth of SP was found to follow an exponential curve.

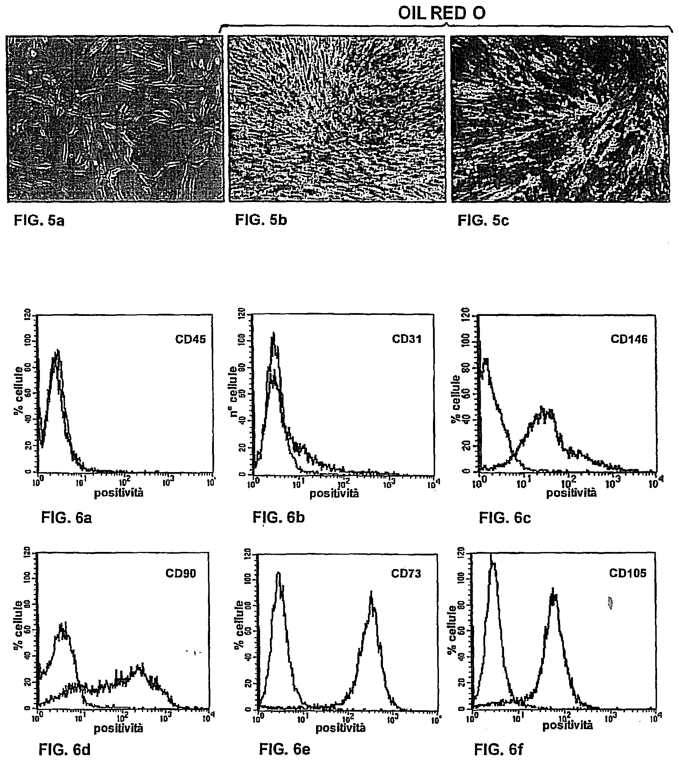

Furthermore, the SP so isolated (FIG. 5a) maintained a typically fibroblastoid phenotype in the culture, and no longer showed the typical intracytoplasmic vacuoles of pre-adipocytes.

Hence, as shown in Figure 5b, the SP were found to be negative for "Oil Red O" staining, which stains intracellular granules. When comparing the SP with the pre-adipocytes isolated from sample P (Fig. 5c), the SP are found to have lost the typical intracytoplasmic lipid vacuoles of pre-adipocytes, and to maintain a fibroblastoid phenotype. This shows conversion of pre-adipocytes to a more undifferentiated stage.

The SP so obtained in the process have been used for later analysis (immunophenotype and differentiation assays), which confirmed the desired characteristics of the population obtained with the method of the invention.

### Example 2: Immunophenotyping evaluation.

First a check was made for the presence of any contaminating population, such as endothelial cells and immune system cells, by searching for their respective CD31 and CD45 markers. The SP were found to be negative for CD45 (FIG. 6a) and very weakly positive for CD31 (FIG. 6b).

At the same time, the SP were found to be positive for progenitor antigens, such as CD90 (FIG. 6d), CD105 (FIG. 6f) and CD73 (FIG. 6e) and to a lesser extent for CD146 (FIG. 6c), as a typical stromal pericyte marker.

### Example 3: Differentiation assays.

After phenotyping, osteogenic, chondrogenic and adopogenic differentiation of the SP was performed, at passage 4.

### Osteogenic differentiation:

The cells were seeded with a density of 10,000 cells/cm². After full growth (typically after 2-4 days), they were induced osteogenic differentiation, with one sample being preserved as a control.

Bone induction was obtained using an appropriate medium, composed of: basal medium (DMEM with 10% fetal calf serum - FCS) dexamethasone, L-ascorbic-2-phosphate acid and β-glycerophosphate.

Such basal medium was maintained for one week, and replaced every 2-3 days. On the seventh day, or day 7, bone morphogenetic protein-2 was added to the medium. The cells were maintained with the differentiating medium for seven more days, and such medium was replaced every two-three days.

On the fourteenth day, the differentiation result was assessed by histological assay (Alizarin Red staining). In this assay, the cells in the flasks are briefly washed in a Tris-HCI and NaCl solution (3-5 mL/flask), fixed with 100% methanol at 4°C for 30 minutes and briefly washed twice in deionized water. Then, the cells are left in contact with a (0.5%) Alizarin Red solution at pH 4.0-4.2 for five minutes and briefly washed.

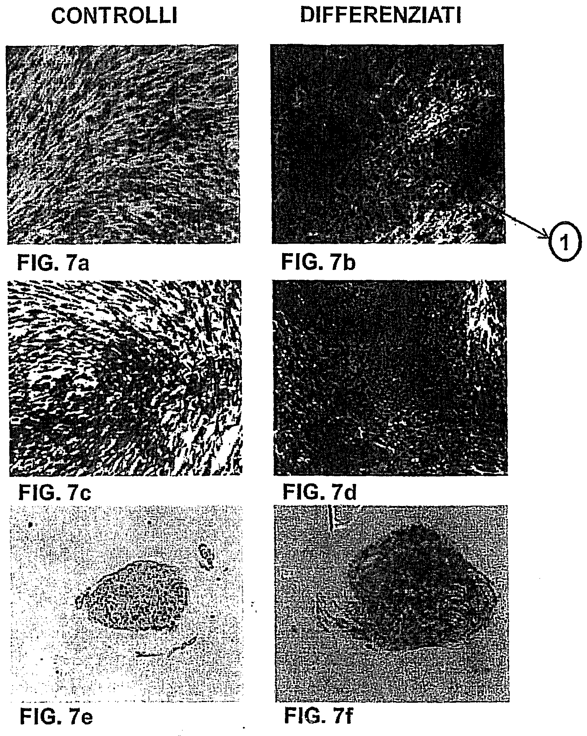

Appropriately induced cells have typical properties of bone tissue osteoblasts, such as production of bone matrix deposits, as observed in zone 1 of FIG. 7b, which are not present in the non-induced control, as shown in FIG. 7a.

### Adipogenic differentiation.

The cells were seeded with a density of 10,000 cells/cm² in their culture medium: once full growth was attained (generally after two-four days), adipogenic differentiation was induced, using one culture as a control.

The only medium for adipogenic differentiation is DMEM added with horse serum and rabbit serum, dexamethasone, insulin, isobutyl methyl xanthine (IBMX), indomethacin and penicillin/streptomycin.

The cells were maintained under differentiating conditions for 10 days, and the medium was replaced every two-three days. On the tenth day, the optical microscope revealed the appearance of characteristic cell clusters, containing lipid vacuoles.

These vacuoles were more apparent by Oil Red O staining: the cells were washed in a saline solution and fixed with 40% formalin fumes for ten minutes.

Then they were washed in ddH₂0 for two minutes, followed by staining with an Oil Red O solution (2%) for five minutes. In order to highlight nuclear and cytoplasmic structures, the cells were treated with hematoxylin as a counterstain (30" - 1'). Finally, they were washed in water for five minutes.

The cells were thus differentiated into adipocytes, as shown by the presence of lipid vacuoles; such presence is further confirmed by their positivity for "Oil Red O" staining (see FIG. 7d) and further validated by the negativity of the control, which only exhibits the purple counterstain of hematoxylin (see FIG. 7c).

### Chondrogenic differentiation.

The cells obtained from the adherent phase are divided into 15 mL conical tubes (2x10⁵ cells/mL) with DMEM high glucose supplemented with BMP-6, TGF-β3, dexamethasone, L-ascorbic-2-phosphate acid, sodium pyruvate, proline, glutamine and penicillin/streptomycin.

The cells were centrifuged to the bottom of 15 mL conical tubes and cultivated, with the medium being changed every two days.

On the twenty-first differentiation day, the pellets were collected, fixed in formalin and included in paraffin.

Serial sections of induced and non-induced samples were then stained with an Alcian Blue solution.

This is a basic water-soluble copper phthalocyanin stain which stains the acidic groups of hyaluronic acid produced by chondro-differentiated cells.

The induced Alcian Blue stained sample assumes a stronger stain (see FIG. 7f) as compared with the non-induced control (see FIG. 7e). Hyaluronic acid is stained, and its production causes a volume increase of the induced sample, when compared with the non-induced sample.

Therefore, the differentiation assays, in combination with immunophenotyping, confirmed the multipotential progenitor characteristics of isolated cells, even when using small amounts of AT.

The invention has been found to fulfill the intended objects.

The invention so conceived is susceptible to changes and variants within the inventive concept.

Furthermore, all the details may be replaced by other technically equivalent elements, as needed, without departure from the scope as defined by the following claims.

## Claims

1. An *in vitro* method for obtaining a population of stromal progenitor cells from a first selected quantity of adipose tissue containing adipose cells , the method comprising the steps of;
- digesting said first quantity with a second quantity of enzymatic buffer of the type used in dissociation of organic and biologic tissues to obtain a population of free cells;
- seeding this population of free cells into culture media needed for their expansion
**characterized in that** said adipose tissue is white adipose tissues of subcutaneous provenience and said first quantity is between 0,004 ml and 0,036 ml and that second quantity is between 0,9 ml and 1,1 ml, and **in that** said second quantity comprises solvent and soluble means which comprise:
Dulbecco's Modified Eagle Medium (DMEM)
Penicillin (1U/ml) & Streptomycin (1mg/ml)
Sodium Pyruvate (1mM) Non-essential amino acids comprising: L-Alanine (0,89 mg/dl); L-Asparagine H₂0 (1,5 mg/dl); L-Aspartic Acid (1,33 mg/dl); L-Glutamic Acid (1,47 mg/dl); Glycine (0,75 mg/dl); L-Proline (1,15 mg/dl); L-Serine (1,05 mg/dl) and wherein said soluble means comprise:
- class I and II Collagenase mixture; and that
- said class I and II Collagenase mixture has an enzymatic activity between 0.1 U/mg and 5.5 U/mg of lyophilized product, having an enzymatic concentration between 1,74 and 1,78 U/ml of said solvent means and wherein
said soluble means comprise:
- Clostripain at a concentration between 0 U/mg and 7.6 U/mg of lyophilized product;
- Proteases at a concentration between 0.05 U/mg and 164 U/mg of liophylized product;
- Trypsin at a concentration between 0.23 U/mg and 2.3 U/mg of liophylized product in a form selected between single agents or in combination.

2. The method according to claim 1, wherein the said digesting comprises to shake with shaking devices said first and second quantities for a time between 20 minutes and 120 minutes, at a pre-established temperature.

3. The method according to claim 2, wherein said pre-established temperature is comprised between 36°C and 38°C.

4. The method according to claim 2, wherein the said to shake comprises to swing said shaking devices in a selected swinging direction according to a first number of swings per minute and in a second swinging direction different from said first swinging direction and according to a second number of swing per minute.

5. The method according to claim 4, wherein said first swinging direction comprises a straight direction and said second swinging direction comprises an orbital direction.

6. The method according to claim 4, wherein the said first number is between 70 and 80 swings per minute and the said second number is between 6 and 100 swings per minute.

7. The method according to claim 1, wherein said stromal progenitor cells comprise mesenchymal stromal cells and stromal pericytes.

## Patentansprüche

1. In-vitro-Verfahren zum Gewinnen einer Population von Vorläuferzellen eines Bindegewebes aus einer ersten ausgewählten Menge von Fettgewebe, das Fettzellen enthält, wobei das Verfahren die folgenden Schritte umfasst:
- Verdauen der ersten Menge mit einer zweiten Menge von enzymatischem Puffer von der Art, die bei der Spaltung von organischem und biologischem Gewebe verwendet wird, um eine Population freier Zellen zu gewinnen;
- Aussäen dieser Population freier Zellen in ein Kulturmedium, das für deren Expansion benötigt wird;
**dadurch gekennzeichnet, dass** das Fettgewebe weißes Fettgewebe subkutanen Ursprungs ist und die erste Menge zwischen 0,004 ml und 0,036 ml beträgt und dass die zweite Menge zwischen 0,9 ml und 1,1 ml beträgt, und dadurch, dass die zweite Menge Lösungsmittel und lösliche Mittel umfasst, die Folgendes umfassen:
Dulbecco's Modified Eagle Medium (DMEM)
Penicillin (1 U/ml) & Streptomycin (1 mg/ml)
Natriumpyruvat (1 mM)
nicht essentielle Aminosäuren, umfassend: L-Alanin (0,89 mg/dl); L-Asparagin-H₂O (1,5 mg/dl); L-Asparaginsäure (1,33 mg/dl); L-Glutaminsäure (1,47 mg/dl); Glycin (0,75 mg/dl); L-Prolin (1,15 mg/dl); L-Serin (1,05 mg/dl), und wobei
die löslichen Mittel Folgendes umfassen:
- ein Kollagenase-Gemisch der Klasse I und II; und dass
- das Kollagenase-Gemisch der Klasse I und II eine enzymatische Aktivität zwischen 0,1 U/mg und 5,5 U/mg des lyophilisierten Produkts hat und eine enzymatische Konzentration zwischen 1,74 und 1,78 U/ml des Lösungsmittelmittels hat, und wobei
die löslichen Mittel Folgendes umfassen:
- Clostripain in einer Konzentration zwischen 0 U/mg und 7,6 U/mg des lyophilisierten Produkts;
- Proteasen in einer Konzentration zwischen 0,05 U/mg und 164 U/mg des lyophilisierten Produkts;
- Trypsin in einer Konzentration zwischen 0,23 U/mg und 2,3 U/mg des lyophilisierten Produkts in einer aus Einzelstoffen oder einer Kombination ausgewählten Form.

2. Verfahren nach Anspruch 1, wobei das Verdauen ein Schütteln der ersten und der zweiten Menge mit Schüttelvorrichtungen für einen Zeitraum zwischen 20 Minuten und 120 Minuten bei einer zuvor festgelegten Temperatur umfasst.

3. Verfahren nach Anspruch 2, wobei die zuvor festgelegte Temperatur zwischen 36 °C und 38 °C enthalten ist.

4. Verfahren nach Anspruch 2, wobei das Schütteln ein Schwingen der Schüttelvorrichtungen in einer ersten Schwingrichtung gemäß einer ersten Anzahl von Schwingbewegungen pro Minute und in einer von der ersten Schwingrichtung unterschiedlichen zweiten Schwingrichtung gemäß einer zweiten Anzahl von Schwingbewegungen pro Minute umfasst.

5. Verfahren nach Anspruch 4, wobei die erste Schwingrichtung eine geradlinige Richtung umfasst und die zweite Schwingrichtung eine Umlaufrichtung umfasst.

6. Verfahren nach Anspruch 4, wobei die erste Anzahl zwischen 70 und 80 Schwingbewegungen pro Minute liegt und die zweite Anzahl zwischen 6 und 100 Schwingbewegungen pro Minute liegt.

7. Verfahren nach Anspruch 1, wobei die Vorläuferzellen des Bindegewebes mesenchymale Bindegewebszellen und Pericyten des Bindegewebes umfassen.

## Revendications

1. Procédé *in vitro* pour obtenir une population de cellules progénitrices stromales à partir d'une première quantité sélectionnée de tissu adipeux contenant des cellules adipeuses, le procédé comprenant les étapes de :
- digestion de ladite première quantité avec une seconde quantité de tampon enzymatique du type utilisé dans la dissociation de tissus organiques et biologiques pour obtenir une population de cellules libres ;
- ensemencement de cette population de cellules libres dans des milieux de culture nécessaires pour leur expansion
**caractérisé en ce que** ledit tissu adipeux est constitué de tissus adipeux blancs de provenance sous-cutanée et ladite première quantité est entre 0,004 ml et 0,036 ml et qu'une seconde quantité est entre 0,9 ml et 1,1 ml, et **en ce que** ladite seconde quantité comprend un moyen solvant et un moyen soluble qui comprennent :
un milieu Eagle modifié par Dulbecco (DMEM)
de la Pénicilline (1 U/ml) et de la Streptomycine (1 mg/ml)
du Pyruvate de Sodium (1 mM)
des acides aminés non essentiels comprenant : L-Alanine (0,89 mg/dl) ; L-Asparagine H₂O (1,5 mg/dl) ; L-Acide Aspartique (1,33 mg/dl) ; L-Acide Glutamique (1,47 mg/dl) ; Glycine (0,75 mg/dl) ; L-Proline (1,15 mg/dl) ; L-Sérine (1,05 mg/dl) et dans lequel
ledit moyen soluble comprend :
- un mélange de Collagénase de classe I et II ; et que
- ledit mélange de Collagénase de classe I et II a une activité enzymatique entre 0,1 U/mg et 5,5 U/mg de produit lyophilisé, ayant une concentration enzymatique entre 1,74 et 1,78 U/ml dudit moyen solvant et dans lequel ledit moyen soluble comprend :
- de la Clostripaïne à une concentration entre 0 U/mg et 7,6 U/mg de produit lyophilisé ;
- des protéases à une concentration entre 0,05 U/mg et 164 U/mg de produit lyophilisé ;
- de la trypsine à une concentration entre 0,23 U/mg et 2,3 U/mg de produit lyophilisé sous une forme sélectionnée entre des agents uniques ou en combinaison.

2. Procédé selon la revendication 1, dans lequel ladite digestion comprend l'agitation avec des dispositifs d'agitation desdites première et seconde quantités pendant un temps entre 20 minutes et 120 minutes, à une température préétablie.

3. Procédé selon la revendication 2, dans lequel ladite température préétablie est comprise entre 36 °C et 38 °C.

4. Procédé selon la revendication 2, dans lequel ladite agitation comprend le basculement desdits dispositifs d'agitation dans une direction de basculement sélectionnée selon un premier nombre de basculements par minute et dans une seconde direction de basculement différente de ladite première direction de basculement et selon un second nombre de basculements par minute.

5. Procédé selon la revendication 4, dans lequel ladite première direction de basculement comprend une direction linéaire et ladite seconde direction de basculement comprend une direction orbitale.

6. Procédé selon la revendication 4, dans lequel ledit premier nombre est entre 70 et 80 basculements par minute et ledit second nombre est entre 6 et 100 basculements par minute.

7. Procédé selon la revendication 1, dans lequel lesdites cellules progénitrices stromales comprennent des cellules stromales mésenchymateuses et des péricytes stromaux.
